# EUROPEAN PATENT APPLICATION

(11) **EP 4 062 829 A1**
(43) Date of publication of application: **28.09.2022**
(21) Application number: 20896079.9
(22) Date of filing: 14.10.2020
(51) Int. Cl.: A61B 5/145, A61B 5/00, A61B 10/00, G01N 33/487

(54) **BODY FLUID OBSERVATION DEVICE**

(30) Priority: 02.12.2019 KR 20190158435; 09.09.2020 KR 20200115123
(71) Applicant: INTIN INC., Daegu 41069 (KR)
(72) Inventor: KIM, Ji Hoon, Seoul 18449 (KR)
(74) Representative: Sander, Rolf
(86) International application number: PCT/KR2020/013992
(87) International publication number: WO 2021/112393

(57) **Abstract**

Provided is a body fluid observation device for observing a user's body fluid.

The body fluid observation device includes: a chamber having a length in a first horizontal direction and having a user's body fluid received therein; and a body into which the chamber is inserted to monitor the body fluid, wherein the body includes a case provided with a chamber insertion hole, into which the chamber is inserted in a front surface of the body, a guide partition extending from the chamber insertion hole in the first horizontal direction to guide the chamber, and an observation hole provided through a bottom surface of the body to allow observation of the body fluid; a light source part positioned above the chamber in the case and emitting light; and an operation part electrically connected to the light source part to operate the light source part, wherein, as the chamber is inserted into the chamber insertion hole, the operation part forms an electric closed loop for operating the light source part.

## Description

### [Technical Field]

The present disclosure relates to a body fluid observation device for observing a user's body fluid.

### [Background Art]

Generally, people should visit a specialized medical institution to check there health state and body state. However, in order to receive diagnosis, people need to set aside time for visiting a specialized medical institution during busy work time, and it takes a complicated procedure.

In particular, this procedure requires a long wait to receive an examination, but the actual examination or inspection is conducted in a very short time. That is, it takes a lot of time and money to receive the examination or inspection, which involves a lot of inconvenience.

Thus, most people ignore endurable discomfort, and visit specialized medical institutions only when having unendurable pain or discomfort.

In order to solve the inconvenience, techniques have been developed wherein various types of advanced equipment are used to frequently check one's body state and the result is transmitted to a specialized medical institution via a network. However, techniques that are commercialized and are actually used in practice are extremely rare.

Preferably, the use of a simple tester, which has been used for a long time, continues and is increasingly used by the younger generation due to ease of use, low cost, and convenience.

Such a tester detects biochemical substances such as hormones discharged under particular circumstances by using secretions from people's bodies, e.g., saliva, urine, sweat, etc. The tester may be used to detect hormones included in the biochemical substances or to check a change in state of the biochemical substances.

The tester uses a change in the composition of body fluid or a change in state when the body is subjected to a specific situation, for example, a specific disease, infection, or abnormality or peculiarity of the body such as ovulation.

### [Disclosure]

### [Technical Problem]

The present disclosure provides a body fluid observation device for observing a user's body fluid.

### [Technical Solution]

In embodiments, a body fluid observation device includes: a chamber having a length in a first horizontal direction and having a user's body fluid received therein; and a body into which the chamber is inserted to monitor the body fluid, wherein the body includes a case provided with a chamber insertion hole, into which the chamber is inserted in a front surface of the body, a guide partition extending from the chamber insertion hole in the first horizontal direction to guide the chamber, and an observation hole provided through a bottom surface of the body to allow observation of the body fluid; a light source part positioned above the chamber in the case and emitting light; and an operation part electrically connected to the light source part to operate the light source part, wherein, as the chamber is inserted into the chamber insertion hole, the operation part forms an electric closed loop for operating the light source part.

The chamber may include a body fluid receiving groove in which a portion for seating the body fluid is received in an upper surface, and, in the chamber, at least the body fluid receiving groove may be transparent or translucent.

In addition, the chamber may have a guide protrusion formed in a lower surface thereof and protruding downward, and the main body may be positioned below the chamber insertion hole and include a guide groove into which the guide protrusion is inserted in the first horizontal direction as the chamber is inserted into the chamber insertion hole.

The guide partition may extend from an end of the first horizontal direction in a second horizontal direction crossing the first horizontal direction, and a shape of the guide partition extending in the second horizontal direction may correspond to a planar shape at an end of the chamber in the first horizontal direction.

In the chamber, the planar shape at the end of the first horizontal direction is asymmetric in both sides thereof with respect to a center line parallel to the first horizontal direction, and a shape of the guide partition extending from the guide partition in the second horizontal direction may be asymmetric in both sides thereof with respect to a center line parallel to the first horizontal direction.

The operation part may include an operation switch to be turned on by being pressed by the chamber while the chamber is inserted into the chamber insertion hole, and to form the electric closed loop.

The operation switch may be located on an extension line of the guide partition.

The operation part may include: a first substrate provided with a circuit pattern electrically connected to the light source part; and a second substrate located under the first substrate, electrically connected to the first substrate, and provided with the operation switch disposed thereunder, and the operation switch may be fixed to overlap on an extension line of the guide partition under the second substrate.

The light source part may be located in a central portion of the first substrate, and a light transmission hole through which light emitted from the light source part passes may be provided in a central portion of the second substrate.

The chamber may include a chamber electrode having an electrode pattern of a conductive material, and the operation part may include a guide electrode located at a portion of the guide partition corresponding to the chamber electrode so as to form the electric closed loop, so that the guide electrode is connected to the chamber electrode upon insertion of the chamber.

The main body may further include a lens part fixedly coupled to the observation hole of the case to enlarge an image of the body fluid.

The body fluid observation device may further include a main body holder fitted to an outer surface of the body so as to fix the body to a device having a camera.

The case of the main body may include a case coupling groove recessed along an outer circumferential surface of the case of the main body so that the main body holder is coupled thereto, and the main body holder may include: a coupling wing portion formed in an arc shape to surround the outer circumferential surface of the case of the main body and to be inserted into the case coupling groove; and a body portion extending from a central portion of the coupling wing portion to be coupled to a user terminal.

The main body may include: a first locking protrusion protruding from a bottom surface of the case coupling groove at both portions adjacent to the chamber insertion hole in the case coupling groove; and a second locking protrusion provided at a portion on an opposite side to the chamber insertion hole in the case coupling groove.

The coupling wing portion may include: a first locking groove provided at both ends of the arc, the first locking groove into which the first locking protrusion is inserted; and a second locking groove provided at a central portion of the arc, the second locking groove into which the second locking protrusion is inserted.

The body holder may include a deformation prevention hole at a portion connected to the body portion in the coupling wing portion.

### [Advantageous Effects]

In a body fluid observation device according to an example of the present disclosure, as a chamber is inserted into a main body through a chamber insertion hole, an operation part may form an electric closed loop for operating a light source part, so that a user can more conveniently observe body fluid.

### [Description of Drawings]

FIG. 1 is a view showing the overall appearance of a body fluid observation device according to an example of the present disclosure.
FIG. 2 is a view for explaining an example in which a main body holder 300 is further coupled to a main body 200 of a body fluid observation device according to an example of the present disclosure.
FIG. 3 is a view for explaining an example in which the body fluid observation device shown in FIG. 2 is coupled to a user terminal 10 and used.
FIG. 4 is a view for explaining an example of a body fluid image observed through the user terminal 10 when the body fluid observation device is used in combination with the user terminal 10.
FIG. 5 is a view for explaining an example of the chamber 100 in the body fluid observation device according to an example of the present disclosure.
FIG. 6 is an exploded view of the main body 200 of the body fluid observation device according to an example of the present disclosure.
FIG. 7 is a vertical cross-sectional view of the main body 200 of the body fluid observation device according to an example of the present disclosure.
FIG. 8 is a view for describing the case 210 in more detail in FIGS. 6 and 7.
FIG. 9 is a view for explaining an asymmetric structure of the chamber 100 and the guide partitions R211x and R211y.
FIG. 10 is a view for explaining in more detail an example of the operation part 220 according to the present disclosure.
FIG. 11 is a view for explaining a method in which the operation part 220 shown in FIG. 9 operates.
FIG. 12 is a view for explaining another example of the operation part 220 according to the present disclosure.
FIG. 13 is a view for explaining a modified example of the chamber according to an example of the present disclosure.
FIG. 14 and 15 are views for explaining a structure in which the coupling holder and the main body are coupled in the body fluid observation device according to an example of the present disclosure.
FIG. 16 is a view for explaining a modified example of the coupling holder shown in FIG.

### [Mode for Disclosure]

Hereinafter, embodiments of the present disclosure will be described in detail with reference to the accompanying drawings. In describing the present disclosure, if it is determined that a detailed description of known functions and components associated with the present disclosure unnecessarily obscure the gist of the present disclosure, the detailed description thereof will be omitted. The terms used henceforth are used to appropriately express the embodiments of the present disclosure and may be altered according to a person of a related field or conventional practice. Therefore, the terms should be defined on the basis of the entire content of this specification.

Hereinafter, in describing a first horizontal direction and a second horizontal direction, when any one direction on a plane is defined as the first horizontal direction, the second horizontal direction refers to a direction crossing or intersecting the first horizontal direction. That is, the second horizontal direction may intersect the first horizontal direction at a right angle, but not necessarily limited thereto, and a range similar to the right angle may be included.

A vertical direction refers to a direction crossing a horizontal plane formed by the first and second horizontal directions. For example, the vertical direction may vertically intersect the first and second horizontal directions, but it is not necessarily limited thereto, and a range similar to a vertical extent may be included.

Hereinafter, a body fluid observation device according to an example of the present disclosure will be described with reference to the accompanying drawings.

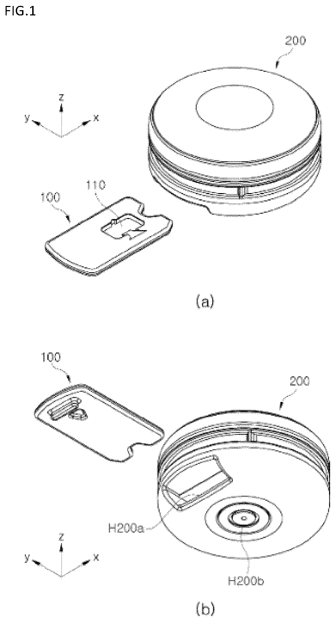

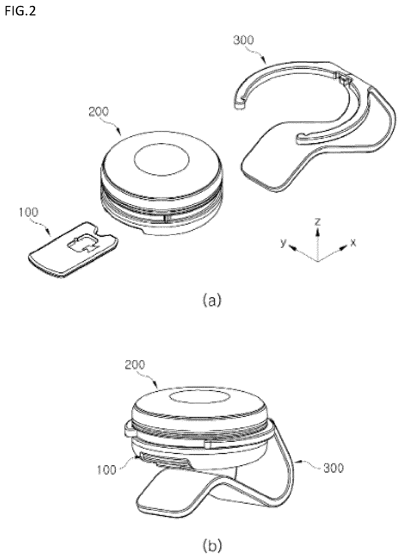

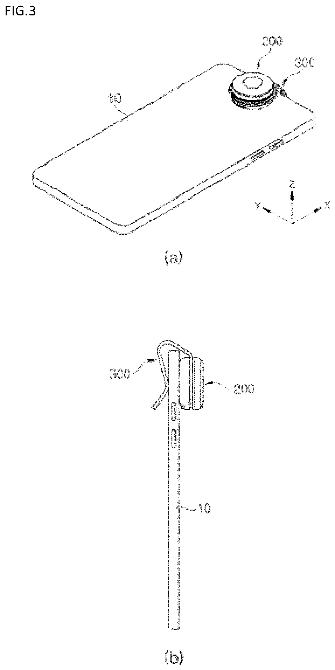

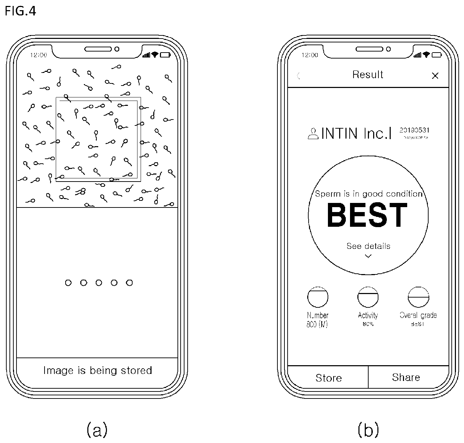

FIG. 1 shows the overall appearance of the body fluid observation device according to an example of the present disclosure, and FIG. 2 illustrates an example in which the main body holder 300 is further coupled to the main body 200 of the body fluid observation device according to an example of the present disclosure, FIG. 3 is a view for explaining an example in which the body fluid observation device shown in FIG. 2 is coupled to the user terminal 10 and used, and FIG. 4 is a body fluid observation device coupled to the user terminal 10. When used, it is a view for explaining an example of a body fluid image observed through the user terminal 10.

As shown in FIG. 1 (a) and FIG. 1 (b), a body fluid observation device according to an example of the present disclosure may include a chamber 100 and a main body 200.

The chamber 100 has a length in the first horizontal direction x, and the user's body fluid may be received in the chamber 100, and to this end, the chamber 100 may be provided with a body fluid receiving groove 110 in which the user's body fluid is to be received.

In order to observe the body fluid, the main body 200 may include a chamber insertion hole H200a into which the chamber 100 is inserted, and an observation hole H200b provided in a bottom surface of the main body 200 to observe the body fluid.

After the chamber 100 having the user's body fluid received therein is inserted into the main body 200 through the chamber insertion hole H200a, the user's body fluid may be observed through the observation hole H200b.

Here, a lens part 250 may be coupled to the observation hole H200b to observe an enlarged image of the body fluid, and the user may be able to observe the user's body fluid through the observation hole H200b with naked eyes.

However, the present disclosure is not necessarily limited thereto, and the body fluid observation device according to an example of the present disclosure may be used in combination with the user terminal 10 provided having a camera.

A body fluid analysis program or application for analyzing video image information acquired through the body fluid observation device may be installed in the user terminal 10.

For an instance, the user terminal 10 may include any wired or wireless communication device capable of communication, and may include a mobile phone, a smart phone, a tablet computer, a laptop computer, a portable media player, a personal digital assistant (PDA), a wearable device including a smart watch or smart glasses capable of wireless communication, a navigation device, and the like.

However, even if the body fluid analysis program or application is not installed in the user terminal 10, video image information acquired by the camera of the user terminal 10 may be transmitted to another electronic communication device in which the body fluid analysis program or application is installed.

To this end, as shown in FIG. 2 (a) and FIG. 2 (b), the body fluid observation device according to an exemplary embodiment of the present disclosure may further include a main body holder 300 capable of being coupled to the user terminal 10.

The body holder 300 may be fitted to an outer surface of the main body 200, as shown in FIG. 2 (b), and may be coupled to an electronic device having a camera, that is, the user terminal 10 to fix the main body 200, as shown in FIG. 3.

As such, as shown in FIG. 3 (a) and FIG. 3 (b), when the body fluid observation device is coupled to the user terminal 10 in which a body fluid analysis program or application is installed, the user's body fluid may be imaged as shown in FIG. 4 (a).

If the user's body fluid is analyzed using the body fluid analysis program or application as shown in FIG. 4 (b), a state or activity of the body fluid may be analyzed, and thus, it is possible to conveniently analyze the user's body fluid without visiting a specialized medical institution, thereby further improving user's convenience and comfort.

Hereinafter, a detailed structure of such a body fluid tester will be described.

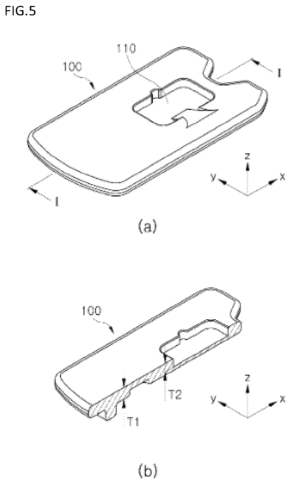

FIG. 5 is a view for explaining an example of the chamber 100 in the body fluid observation device according to an example of the present disclosure.

FIG. 5 (b) shows a cross-section taken along line II of (a) FIG. 5 (a). As shown in FIGS. 5 (a) and FIG. 5 (b), the chamber 100 has a length in the first horizontal direction x and may include a body fluid receiving groove 110 in which a user's body fluid is received. The body fluid receiving groove 110 may be formed by being recessed inwardly from an upper surface of the chamber 100.

Here, a recess depth T2 of the body fluid receiving groove 110 may be greater than 1/2 of a chamber thickness T1 and may be less than 9/10 of the chamber thickness T1.

In order to allow body fluid to be evenly spread in the body fluid receiving groove 110 using capillary action after the body fluid is received in the body fluid receiving groove 110, a separate insertion member (not shown) to be inserted into the body fluid receiving groove 110 may be further provided.

In such a chamber 100, the body fluid receiving groove 110 may be at least transparent or translucent. For an instance, only the body fluid receiving groove 110 in the chamber 100 may be transparent or translucent whereas the others are opaque, or the entire chamber 100 including the body fluid receiving groove 110 may be transparent or translucent. Accordingly, the user's body fluid received in the body fluid receiving groove 110 may be easily observed from a rear side of the body fluid receiving groove 110.

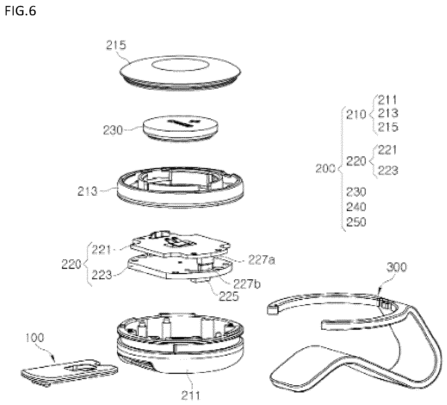

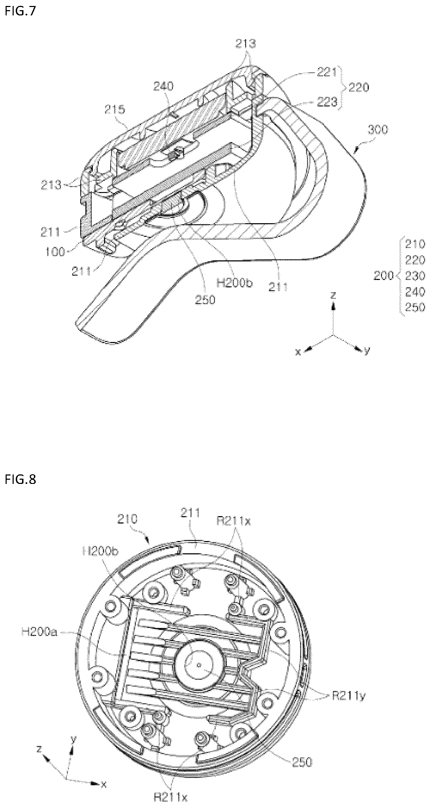

In order to observe the user's body fluid, the main body 200 in which the chamber 100 having the user's chamber 100 received therein is inserted may be formed, for example, as shown in FIGS. 6 and 7.

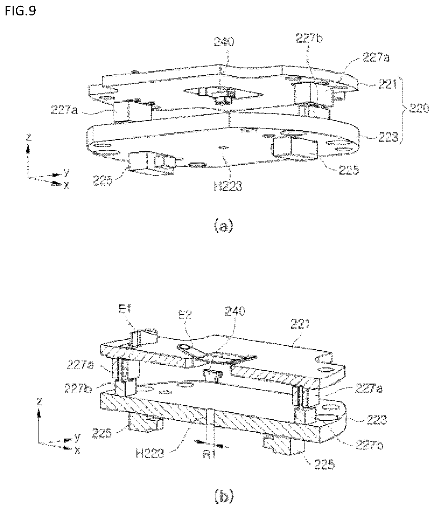

FIG. 6 is an exploded view of the main body 200 of the body fluid observation device according to an example of the present disclosure, FIG. 7 is a vertical cross-sectional view of the main body 200 of the body fluid observation device according to an example of the present disclosure, FIG. 8 is a view for explaining a case shown in FIGS. 6 and 7 in more detail, and FIG. 9 is a view for explaining an asymmetric structure of the chamber 100 and a guide partition R211x and R211y.

As shown in FIGS. 6 and 7, the main body 200 of the body fluid observation device according to an example of the present disclosure may include the case 210, an operation part 220, a power supply part 230, a light source part 240, and a lens part 250.

As shown in FIGS. 6 and 7, the case 210 may include first, second, and third case parts 211, 213, and 215, and the first case part 211 may be located at a bottom of the main body 200, the second case part 213 may be located in the middle of the main body 200, and the third case part 215 may be located at a top of the main body 200.

As shown in FIG. 1, a space in which the chamber 100 can be inserted, a space in which the light source part 240 and the operation part 220 can be located, and the lens part 250 may be provided in the first case part 211, and the second case part 213 may be located above the first case 211 to cover the operation part 220 so that the space in which the chamber 100 is to be inserted and the space in which the light source part 240 and the operation part 220 are located can be hidden and protected and a position of a battery can be fixed.

The third case part 215 may be located above the second case part 213 to cover the power supply part 230 and may be detachable from the second case part 213 so that the user can easily replace the battery.

Here, the first case part 211 may include a chamber insertion hole H200a, a guide partition R211x and R211y, and an observation hole H200b, as shown in FIG. 8.

The chamber insertion hole H200a is located at a front side of the main body 200 through which the chamber 100 is inserted, so that a portion of the first case part 210 is opened to have a length in the second horizontal direction y, thereby allowing the chamber 100 to be inserted.

The guide partition R211x and R211y may be located at a bottom surface of the first case part 211, and may extend in the first horizontal direction x from the chamber insertion hole H200a to guide an insertion direction and a position of the chamber 100.

The guide partition R211x and R211y may include a first guide partition R211x extending in the first horizontal direction x and a second guide partition R211y extending in the second horizontal direction y from an end of the first horizontal direction x.

Here, a shape of the second guide partition R211y may correspond to a planar shape at an end of the chamber 100 in the first horizontal direction x. That is, in the chamber 100 of FIG. 5, a portion adjacent to a center line II of the chamber 100 at the end in the first horizontal direction x may have a recessed portion and a planar shape to be inclined diagonally relative to the first and second horizontal directions (x, y) may be provided, and, as shown in FIG. 8, a shape of the second guide partition R211y may also have a portion recessed inward at the end with respect to the center line in the first horizontal direction x and a planar shape to be inclined diagonally relative to the first and second horizontal directions may be provided.

Accordingly, the guide partition R211x and R211y may be able to more accurately guide the position of the chamber 100 in the first and second horizontal directions.

Accordingly, the guide partitions R211x and R211y may be able to guide the chamber 100 so that, when the chamber 100 is inserted into the main body 200 through the chamber insertion hole H200a, the chamber 100 can be accurately positioned at a desired part and the body fluid receiving groove 110 of the chamber 100 can be accurately positioned on the observation hole H200b.

In addition, each of the chamber 100 and the guide partition R211x and R211y according to an example of the present disclosure may have an asymmetric shape with respect to the center line as shown in FIG. 9.

That is, as shown in (a) of FIG. 9, in the chamber 100, the planar shape of the end of the first horizontal direction x may be formed to be asymmetric in both sides thereof with respect to a center line CL100 parallel to the first horizontal direction x, and, in the guide partition R211x and R211y, a shape of the second guide partition R211y extending in the second horizontal direction y may be asymmetric in both sides thereof with respect to a center line CL211 parallel to the first horizontal direction x.

For an instance, the chamber 100 may have a chamfered portion P100 formed diagonally relative to the first and second horizontal directions at an end in the first horizontal direction x, the chamfered portion P1 which is a corner on any one of both sides with respect to the center line CL100.

In addition, as shown in FIG. 9 (a), the guide partition R211x and R211y may also has a chamfered partition PR211 diagonally formed in each of the first and second guide partitions R211x and R211y at an end in the first horizontal direction so that a corner on any one of both sides with respect to the center line CL211 corresponds to the shape of the chamfered portion P100 of the chamber 100.

Accordingly, as shown in (b) and (c) of FIG. 9, only when the chamber 100 is inserted in an intended or desired direction, the chamber 100 may be completely inserted into the main body 200, and when the chamber 100 is inserted in a different direction than intended or desired, the chamber 100 may not be completely inserted into the main body 200.

Therefore, when the chamber 100 is inserted upside down, the chamber 100 may be prevented from being completely inserted into the main body 200. Accordingly, it is possible to further enhance user convenience.

As shown in FIG. 8, the observation hole H200b may be formed at the bottom surface of the first case part 211 to observe body fluid and thus provided at the bottom surface of the main body 200, and the observation hole H200b may be overlapped with the body fluid receiving groove 100 of the chamber 100 in the vertical direction z.

In order to observe the body fluid more precisely, the observation hole H200b may be provided with the lens part 250 that is fixedly coupled to the observation hole H200b, as shown in FIG. 8, to enlarge an image of the body fluid.

The magnification of the lens part 250 may be, for example, 100 to 500 times, and the lens part 250 may be coupled to a camera provided in the user terminal 10 to enlarge body fluid received in the chamber 100 at magnification of 700 to1300 times. However, the present disclosure is not necessarily limited to the above-described magnification of the lens part 250.

As shown in FIG. 7, the light source part 240 may be located above the body fluid receiving groove 110 of the chamber 100 and emit light to the body fluid receiving groove 110. Accordingly, the user may be able to observe the body fluid more clearly.

As shown in FIG. 7, the operation part 220 may be located in an upper portion of the chamber 100 in the first case part 211 and may be electrically connected to the light source part 240 to operate the light source part 240.

The operation part 220 may include a substrate 221 and 223 provided with an electrical circuit pattern for operating the light source part 240, and the light source part 240 may be located at the center of the substrate 221 and 223 while located above the body fluid seating portion of the chamber 100.

As the chamber 100 is inserted into the chamber insertion hole H200a, the operation part 220 may form an electric closed loop for operating the light source part 240, and accordingly, the operation part 220 may operate the light source part 240 so that light can be lighted from the light source part 240.

Accordingly, the user may be able to more clearly observe the body fluid located in the body fluid receiving groove 110 of the chamber 100 through the observation hole H200b with the naked eye or through the user terminal 10.

In order to form a structure of forming an electric closed loop for operating the light source part 240 when the operation part 220 inserted into the chamber 100, a first substrate 221 and a second substrate 223 may be provided in multiple layers.

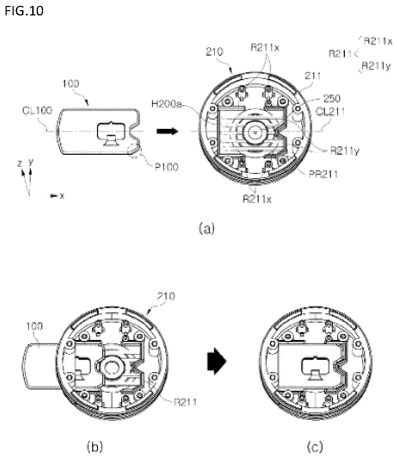

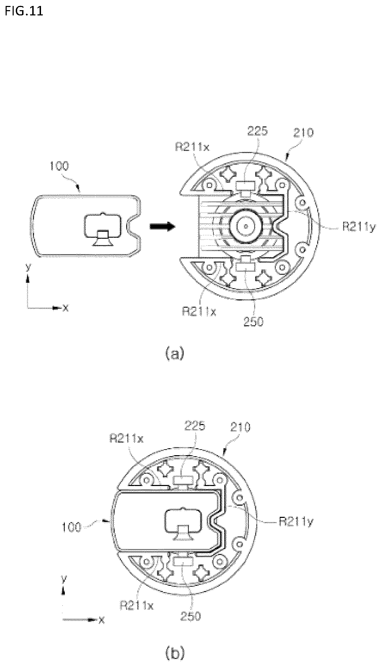

The first substrate 221 may be provided with a circuit pattern electrically connected to the light source part 240, and the light source part 240 may be located in the middle of the first substrate 221, the second substrate 223 may be located under the first substrate 221 and may be electrically connected to the first substrate 221, and an operation switch 225 to form an electric closed loop upon insertion of the chamber 100 may be provided. This will be described in more detail with reference to FIGS. 10 and 11.

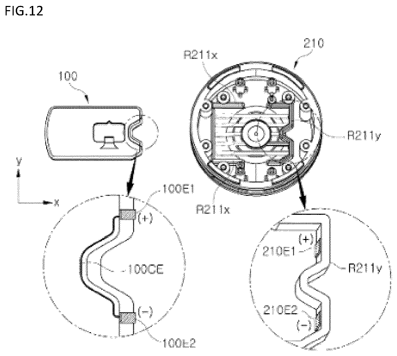

FIG. 10 is a view for explaining in more detail an example of the operation part 220 according to the present disclosure, FIG. 11 is a view for explaining a method of operating the operation part 220 shown in FIG. 9, and FIG. 12 is a view for explaining another example of the operation part 220 according to the present disclosure.

FIG. 10 (a) shows a perspective view of the operation part 220, and FIG. 10 (b) shows a cross-section of the operation part 220, shown in FIG. 10 (a), which is taken along in the second horizontal direction y.

As shown in FIG. 10, an example of the operation part 220 according to the present disclosure may include an operation switch 225 that forms an electric closed loop for operating the light source part 240 when the chamber 100 is inserted into the chamber insertion hole H200a.

More specifically, the operation part 220 according to an example of the present disclosure may be formed in multiple layers, and may include a first substrate 221 located at an upper side and a second substrate 223 positioned at a lower side.

The first substrate 221 may have a light source part 240 positioned in a central portion thereof, and a power supply connection terminal E1 and E2 to which a battery of the power supply part is connected may be provided at the first substrate 221.

In a central portion of the second substrate 223, a light transmission hole H223 through which the light emitted from the light source part 240 passes may be provided, so that light emitted from the light source part 240 located thereabove can transmit. A diameter R1 of the light transmission hole may be, for example, 0.5 to 2.5mm, but this is merely an example, and the diameter R1 of the light transmission hole may vary depending on a distance between the light source part 240 and the light transmission hole H223.

In addition, the first substrate 221 and the second substrate 223 may be electrically connected to each other. For example, a first connector 227a may be provided under the first board 221, a second connector 227b may be provided on the second board 223, and the first substrate 221, and as the second connector 227b is inserted into the first connector 227a, the second substrate 223 may be electrically connected to each other.

The operation switch 225 forming an electric closed loop may be provided under the second substrate 223, and each operation switch 225 may have a switch terminal protruding toward the light transmission hole H223 located in a central portion of the second substrate 223.

Here, the operation switch 225 may be located on an extension line of the guide partition R211x and R211y.

That is, the operation switch 225 may be located on an extension line of the first guide partition R211x extending in the first horizontal direction x, for example, as shown in FIG. 11. The switch terminal of the operation switch 225 may protrude inwardly than the extension line of the guide partition R211x and R211y, so that the switch terminal of the operation switch 225 can be pressed by the chamber upon insertion of the chamber 100.

However, the present disclosure is not necessarily limited thereto, and the operation switch 225 may be located on an extension line of the second guide partition R211y extending in the second horizontal direction y. That is, it is also possible that the operation switch 225 is located on the side of the second guide partition R211y so as to contact an end of the chamber 100 in the first horizontal direction x.

Accordingly, as shown in FIG. 11(a) and FIG. 11(b), the operation switch 225 provided in the second substrate 223 may be turned on by being pressed by the chamber 100 while the chamber 100 is inserted into the chamber insertion hole H200a, so that an electric closed loop for operating the light source part 240 can be formed.

However, the operation part 220 of the present disclosure is not necessarily limited to the structure including the operation switch 225 as shown in FIGS. 10 and 11, and may have other structures.

That is, the operation part 220 according to another example of the present disclosure may have a structure in which an electric closed loop is formed together with the chamber 100 when the chamber 100 is inserted into the chamber insertion hole H200a.

To this end, as shown in FIG. 12, the chamber 100 may include a chamber electrode 100E1 and 100E2 having an electrode pattern of a conductive material at an end in the first horizontal direction x.

That is, the chamber electrodes 100E1 and 100E2 may include first and second chamber electrodes 100E1 and 100E2 formed in the vertical direction z at the end of the first horizontal direction x in the chamber 100, the first and second chamber electrodes 100E1 and 100E2 may be spaced apart from each other in the second direction, and the first and second chamber electrodes 100E1 and 100E2 may be electrically connected to each other by a chamber electrode line 100CE.

In addition, in order to form an electric closed loop, the operation part 220 may include a guide electrode 210E1 and 210E2 located at a portion of the guide partitions R211x and R211y corresponding to the chamber electrode 100E1 and 100E2, so that the guide electrode 210E1 and 210E2 can be connected to the chamber electrode 100E1 and 100E2 upon insertion of the chamber 100.

Here, in the guide partition R211x and R211y, a position corresponding to the chamber electrodes 100E1 and 100E2 may be, for example, an inner side of the second guide partition R211y, and the guide electrode 210E1, 210E2 may be formed in the vertical direction z to the second guide partitions R211y.

The guide electrode 210E1 and 210E2 may be electrically connected to a circuit pattern of the substrate 221 and 223 included in the operation part 220.

Accordingly, when the chamber 100 is inserted into the main body 200, the guide electrode 210E1 and 210E2 provided in the guide partition R211x and R211y and the chamber electrode 100E1 and 100E2 provided in the chamber 100 may contact each other, thereby forming an electric closed loop for operating the light source part 240. Therefore, in order to observe the body fluid, the user may simply insert the chamber 100 into the main body 200 so as to control light to be emitted from the light source unit 240, thereby enabled to observe body fluid more conveniently.

Meanwhile, the structure of the chamber 100 described with reference to FIG. 5 is a structure in which, when the chamber 100 is inserted into the chamber insertion hole H200a of the main body 200, the chamber 100 can be inserted even with the upper and lower sides inverted. In this case, the body fluid cannot be observed properly. When the chamber 100 is inserted into the chamber insertion hole H200a with the upper and lower surfaces of the chamber 100 inverted, body fluid may flow out of the body fluid receiving groove 110 of the chamber 100 and contaminate the surface of the lens part 250, thereby obstructing the user's observation of the body fluid.

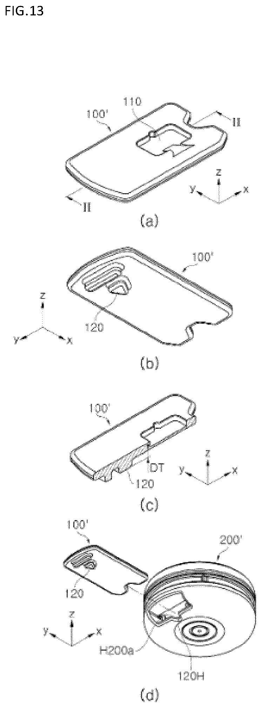

Accordingly, a structure of the chamber 100 improved more than that of FIG. 5 will be described with reference to FIG. 13.

Accordingly, a structure of the chamber improved more than that of FIG. 5 will be described with reference to FIG. 13.

FIG. 13 is a view for explaining a modified example of a chamber 100' according to an example of the present disclosure. FIG. 13 (a) shows an upper surface of the chamber 100' according to the modified example, FIG. 13 (b) shows a lower surface of the chamber 100' according to the modified example, FIG. 13 (c) shows a cross-section taken along II-II line in FIG. 13 (a), and FIG. 13 (d) is a modified example of a case of a main body 200, which is modified to allow the chamber 100' can be inserted.

As shown in FIG. 13 (a), the chamber 100' according to another example of the present disclosure may have the same upper surface structure as that of the chamber 100' according to the example of the present disclosure described with reference to FIG. 5

However, as shown in FIG. 13 (b), the chamber 100' according to another example of the present disclosure may include a guide protrusion 120 protruding downward from a lower surface thereof. A protrusion length DT of the guide protrusion 120 may be identical to a thickness T1 of a body portion of the chamber 100' from the lower surface of the chamber 100', for example, as shown in FIG. 13 (c). That is, a thickness of a portion where the guide protrusion 120 is formed may be about twice a thickness of a body portion of the chamber 100'.

However, the present disclosure is not necessarily limited thereto, and the protrusion length DT of the guide protrusion 120 may vary as long as a thickness (T1+TD) of a portion where the guide protrusion 120 is formed is greater than a height of the chamber insertion hole H200a, so that when the chamber 100' is inserted into the chamber insertion hole H200a, the chamber insertion hole H200a can be caught by the guide protrusion 120 to prevent the chamber 100' from being inserted any longer.

The guide protrusion 120 is illustrated as an example in the form of a triangular prism, but not necessarily limited thereto, and the guide protrusion 120 may take the shape of a cylindrical or polygonal prism.

As such, when the guide protrusion 120 is provided in the chamber 100', the case of the main body 200 may also be modified to have a guide groove 120H into which the guide protrusion 120 of the chamber 100' is inserted.

Specifically, the guide groove 120H is located below the chamber insertion hole H200a, and when the chamber 100' is completely inserted into the chamber insertion hole H200a, the guide protrusion 120 of the chamber 100' may be inserted in the first horizontal direction x. Here, a shape of the guide groove 120H may be formed to correspond to a shape of the guide protrusion 120.

As such, the chamber 100' and the main body 200 according to the modified example of the present disclosure may physically prevent insertion of the chamber 100'when the user tries to insert the chamber 100' upside down into the main body 200, and thus, it is possible to induce the user to correctly use the body fluid observation device and to prevent the surface of the lens part 250 in the main body 200 from being contaminated.

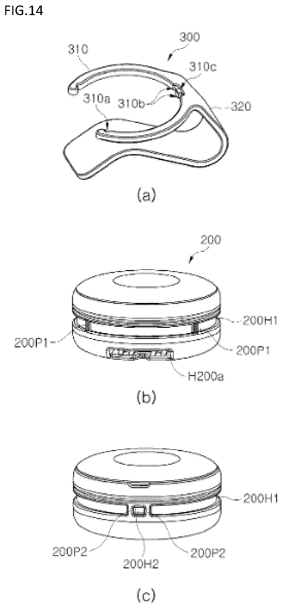

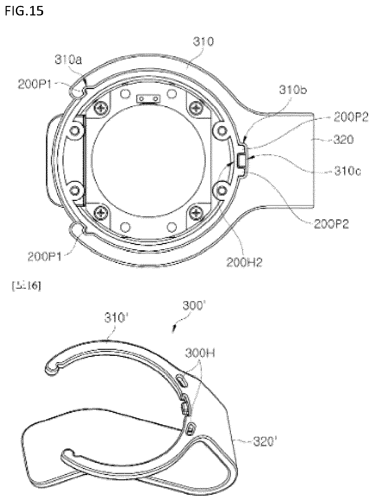

FIGS. 14 and 15 are views for explaining a structure in which the main body holder 300 and the main body 200 are coupled in the body fluid observation device according to an example of the present disclosure, and FIG. 14 (a) is a view for explaining an example of the main body holder 300 coupled to the body 200, and FIG. 14 (b) and FIG. 14 (c) are views for explaining an outer shape of the case to which the main body holder 300 can be more rigidly and precisely coupled when the main body holder 300 is coupled to the body 200. Specifically, FIG. 14 (b) shows a front side of the case, FIG. 14 (c) shows a rear side of the case, and FIG. 15 shows a cross-section in which the main body 200 and the main body holder 300 are coupled to each other.

As shown in FIG. 14 (b) and FIG. 14 (c), the case of the main body 200 may include a case coupling groove 200H1 recessed along an outer circumferential surface to allow coupling with the main body holder 300, so that a coupling wing portion 310 of the main body holder 300 is coupled.

The case coupling groove 200H1 may be recessed into the case of the main body 200 and may extend along the outer circumferential surface with a predetermined width.

Here, as shown in FIG. 14 (b), a first locking protrusion 200P1 protruding from a bottom surface of the case coupling groove 200H1 may be provided in both portions adjacent to the chamber insertion hole H200a in the case coupling groove 200H1, and as shown in FIG. 14 (c), second locking protrusions 200P2 may be provided at a rear side located opposite to the chamber insertion hole H200a in the case coupling groove 200H1. Here, a recessed groove 200H2 may be provided between the second locking protrusions 200P2.

The body holder 300 may include the coupling wing portion 310 and a body portion 320, as shown in FIG. 14 (a). The coupling wing portion 310 may be formed in an arc shape to surround the outer circumferential surface of the case and may be inserted into the case coupling groove 200H1, and the body portion 320 may extend from a central portion of the coupling wing portion 310 to be coupled to a user terminal. Such a coupled state is shown in FIG. 3.

Here, the coupling wing portion 310 may be formed in a circular arc shape and have elasticity, and the circular arc shape may have a longer length than that of a semicircle, so that the main body 200 can be fitted into the arc of the coupling wing portion.

The coupling wing portion 310 may include a first locking groove 310a and a second locking groove 310b, the first locking groove 310a may be located at both ends of the arc of the main body 200 so that the first locking protrusion 200P1 can be inserted, and the second locking groove 310b may be located at a central portion of the arc so that the second locking protrusion 200P2 of the main body 200 can be inserted. Here, a separate protrusion 310c may be provided inside the second locking groove 310b.

Accordingly, in a case where the main body holder 300 is coupled to the main body 200, when the case of the main body 200 is fitted into the arc of the coupling wing 310, as shown in FIG. 15, the first locking protrusion 200P1 of the main body 200 may be inserted into the first locking groove 310a of the coupling wing portion 310 and the second locking protrusion 200P2 of the main body 200 may be inserted into the second locking groove 310b of the coupling wing portion 310, so that the main body 200 can be in closer contact with and more firmly fixed to the coupling wing portion 310 of the main body holder 300.

FIG. 16 is a view for explaining a modified example of the coupling holder shown in FIG.

The body holder 300 according to the modified example of the present disclosure may have a deformation preventing hole 300H in a portion connected to the body 320 in the coupling wing 310. The deformation prevention hole 300H may be formed to have a length in a direction parallel to the arc of the coupling wing and may pass through the coupling wing portion 310.

The deformation prevention hole 300H may prevent deformation of the main body holder 300 caused by a difference in thermal expansion coefficient when the main body holder 300 is manufactured, and the deformation prevention hole 300H may also solve the problem that the main body 200 is not firmly fixed and is easily separated from the coupling wing portion 310 when the main body 200 is fitted even if the main body holder is manufactured without deformation.

Specifically, the main body holder 300 may be manufactured by an injection molding method, and a difference in thermal expansion coefficient occurs due to a difference in thickness and width between the coupling wing portion 310 and the body portion 320, which may cause the main body holder 300 to be easily deformed during the manufacturing process. The deformation prevention hole 300H may reduce the difference in thermal expansion coefficient between the coupling wing portion 310 and the body portion 320, thereby preventing deformation of the main body holder 300.

In addition, even in a case where the coupling wing portion 310 is manufactured without deformation, the entrance of the coupling wing portion 310 may be formed to be opened and closed when the main body 200 is inserted into the coupling wing portion 310 and, in this case, in a case where the deformation preventing hole 300H is provided as in the present disclosure, the deformation prevention hole 300H physically alleviates pressure to the coupling wing portion 310 upon insertion of the main body 200, so that the main body 310 can be more easily fitted and coupled to the coupling wing portion 310 without damage to the coupling wing portion 310.

The present disclosure is not limited to the above-described embodiments and the accompanying drawings, and various modifications and changes may be made in view of a person skilled in the art to which the present disclosure pertains. Therefore, the scope of the present disclosure should be determined by the scope of the appended claims, and equivalents thereof.

## Claims

1. A body fluid observation device comprising:
a chamber having a length in a first horizontal direction and having a user's body fluid received therein; and
a body into which the chamber is inserted to monitor the body fluid,
wherein the body comprises:
a case provided with a chamber insertion hole, into which the chamber is inserted in a front surface of the body, a guide partition extending from the chamber insertion hole in the first horizontal direction to guide the chamber, and an observation hole provided through a bottom surface of the body to allow observation of the body fluid;
a light source part positioned above the chamber in the case and emitting light; and
an operation part electrically connected to the light source part to operate the light source part,
wherein, as the chamber is inserted into the chamber insertion hole, the operation part forms an electric closed loop for operating the light source part.

2. The body fluid observation device of claim 1,
wherein the chamber comprises a body fluid receiving groove in which a portion for seating the body fluid is received in an upper surface, and
wherein in the chamber, at least the body fluid receiving groove is transparent or translucent.

3. The body fluid observation device of claim 2,
wherein the chamber has a guide protrusion formed in a lower surface thereof and protruding downward, and
wherein the main body is positioned below the chamber insertion hole and includes a guide groove into which the guide protrusion is inserted in the first horizontal direction as the chamber is inserted into the chamber insertion hole.

4. The body fluid observation device of claim 1,
wherein the guide partition extends from an end of the first horizontal direction in a second horizontal direction crossing the first horizontal direction, and
wherein a shape of the guide partition extending in the second horizontal direction corresponds to a planar shape at an end of the chamber in the first horizontal direction.

5. The body fluid observation device of claim 3,
wherein in the chamber, the planar shape at the end of the first horizontal direction is asymmetric in both sides thereof with respect to a center line parallel to the first horizontal direction, and
wherein a shape of the guide partition extending from the guide partition in the second horizontal direction is asymmetric in both sides thereof with respect to a center line parallel to the first horizontal direction.

6. The body fluid observation device of claim 1, wherein the operation part comprises an operation switch to be turned on by being pressed by the chamber while the chamber is inserted into the chamber insertion hole, and to form the electric closed loop.

7. The body fluid observation device of claim 6, wherein the operation switch is located on an extension line of the guide partition.

8. The body fluid observation device of claim 6, wherein the operation part comprises:
a first substrate provided with a circuit pattern electrically connected to the light source part; and
a second substrate located under the first substrate, electrically connected to the first substrate, and provided with the operation switch disposed thereunder,
wherein the operation switch is fixed to overlap on an extension line of the guide partition under the second substrate.

9. The body fluid observation device of claim 8, wherein the light source part is located in a central portion of the first substrate, and a light transmission hole through which light emitted from the light source part passes is provided in a central portion of the second substrate.

10. The body fluid observation device of claim 1, wherein the chamber comprises a chamber electrode having an electrode pattern of a conductive material, and the operation part comprises a guide electrode located at a portion of the guide partition corresponding to the chamber electrode so as to form the electric closed loop so that the guide electrode is connected to the chamber electrode upon insertion of the chamber.

11. The body fluid observation device of claim 1, wherein the main body further comprises a lens part fixedly coupled to the observation hole of the case to enlarge an image of the body fluid.

12. The body fluid observation device of claim 1, further comprising:
a main body holder fitted to an outer surface of the body so as to fix the body to a device having a camera.

13. The body fluid observation device of claim 12,
wherein the case of the main body comprises a case coupling groove recessed along an outer circumferential surface of the case of the main body so that the main body holder is coupled thereto, and
wherein the main body holder comprises:
a coupling wing portion formed in an arc shape to surround the outer circumferential surface of the case of the main body and to be inserted into the case coupling groove; and
a body portion extending from a central portion of the coupling wing portion to be coupled to a user terminal.

14. The body fluid observation device of claim 13, wherein the main body comprises a first locking protrusion protruding from a bottom surface of the case coupling groove at both portions adjacent to the chamber insertion hole in the case coupling groove, and a second locking protrusion provided at a portion on an opposite side to the chamber insertion hole in the case coupling groove.

15. The body fluid observation device of claim 14, wherein the coupling wing portion comprises a first locking groove provided at both ends of the arc, the first locking groove into which the first locking protrusion is inserted, and a second locking groove provided at a central portion of the arc, the second locking groove into which the second locking protrusion is inserted.

16. The body fluid observation device of claim 13, wherein the body holder comprises a deformation prevention hole at a portion connected to the body portion in the coupling wing portion.
